# EUROPÄISCHE PATENTSCHRIFT

(11) **EP 4 591 891 B1**
(45) Veröffentlichungstag und Bekanntmachung des Hinweises auf die Patenterteilung: **02.09.2026**
(21) Anmeldenummer: 25152914.5
(22) Anmeldetag: 20.01.2025
(51) Int. Cl.: A61L 2/07, A61B 90/70, A61L 2/26, G01S 19/00

(54) **STERILISIERBARE VORRICHTUNG ZUR ORTUNG VON STERILGUT**
STERILIZABLE DEVICE FOR LOCATING STERILE GOODS
DISPOSITIF STÉRILISABLE POUR LA LOCALISATION D'ARTICLES STÉRILES

(30) Priorität: 24.01.2024 DE 102024101996
(43) Veröffentlichungstag der Anmeldung: 30.07.2025
(73) Patentinhaber: Aesculap AG, 78532 Tuttlingen (DE)
(72) Erfinder: Schweizer, Matthias, 78532 Tuttlingen (DE); Elisch, Andreas, 78655 Dunningen (DE); Högerle, Roland-Alois, 78532 Tuttlingen (DE); Lenzenhuber, Frederick, 78532 Tuttlingen (DE)
(74) Vertreter: DREISS Patentanwälte PartG mbB

(56) Entgegenhaltungen:
- DE-A1- 102021 100 634
- US-A1- 2021 113 729

## Beschreibung

Die Erfindung betrifft ein Verfahren und eine sterilisierbare Vorrichtung zur Ortung von Sterilgut. Die Erfindung betrifft ferner einen Sterilgutbehälter.

US 2021/0113729 A1 offenbart bereits eine Vorrichtung gemäß dem Oberbegriff von Anspruch 1. DE 10 2021 100 634 A1 offenbart ein Gerät zur Verfolgung und Lokalisierung medizinischer Instrumente in einem Sterilisationsprozess. Eine Batterie und eine Schaltung sind in einem gemeinsamen Gehäuse angeordnet.

Die Erfindung geht von einer sterilisierbaren Vorrichtung zur Ortung von Sterilgut aus. Eine derartige Vorrichtung, insbesondere eine Energiequelle der Vorrichtung, soll über eine möglichst lange Lebensdauer verfügen und ist gegen die hohen Temperaturen, die in einem Vorgang zum Waschen oder Sterilisieren auftreten, sowie gegen Wasser oder überhitzten gesättigten Wasserdampf, der beim Vorgang zu Feuchtigkeit auf der Vorrichtung führen kann, sowie gegen auftretende Druckwechsel und einen Druckbereich von 30 mbar absolut bis ~3,1bar absolut während des Sterilisationsvorgangs zu schützen.

Die erfindungsgemäße sterilisierbare Vorrichtung ist gemäß den Merkmalen von Anspruch 1 ausgeführt.

Vorzugsweise ist die Steuerung ausgebildet, eine Temperatur zu erfassen und zumindest den Teil der Elektronik bei einem Erreichen oder Überschreiten eines vorgegebenen Temperaturschwellwerts oder eines insbesondere über eine Schnittstelle der Vorrichtung zur Einstellung der Temperatur vorgebbaren Temperaturschwellwerts zu deaktivieren.

Vorzugsweise ist die Steuerung ausgebildet, zumindest den Teil der Elektronik bei einem Erreichen oder Unterschreiten eines vorgegebenen Temperaturschwellwerts oder eines insbesondere über eine Schnittstelle der Vorrichtung zur Einstellung der Temperatur vorgebbaren Temperaturschwellwerts zu aktivieren.

Vorzugsweise ist der aktive Teil der Elektronik ausgebildet die Temperatur zu erfassen, wobei die Steuerung ausgebildet ist, den deaktivierten Teil der Elektronik in insbesondere regelmäßigen zeitlichen Abständen zu aktivieren, zu prüfen, ob die Temperatur den Temperaturschwellwert unterschreitet, und zumindest den Teil der Elektronik zu deaktivieren, wenn die Temperatur den Temperaturschwellwert erreicht oder überschreitet oder den deaktivierten Teil der Elektronik anderenfalls aktiviert zu lassen.

Vorzugsweise ist die Steuerung ausgebildet, zumindest den Teil der Elektronik für eine insbesondere in einer Firmware der Steuerung vorgegebene Zeitspanne oder eine insbesondere über eine Schnittstelle, insbesondere eine Datenkommunikationsschnittstelle, oder ein mechanisches Element, vorzugsweise einen Schalter oder Drehregler, der Vorrichtung zur Einstellung der Zeitspanne vorgebbare Zeitspanne nach dem Erkennen des Vorgangs zum Sterilisieren deaktiviert zu lassen, und den deaktivierten Teil der Elektronik nach Ablauf der Zeitspanne, in welcher zumindest der Teil der Elektronik deaktiviert zu lassen ist, in insbesondere regelmäßigen zeitlichen Abständen zu aktivieren.

In einer Ausführungsform der Vorrichtung umfasst die Vorrichtung eine Schnittstelle zur Signalkommunikation, die ausgebildet ist, ein Signal zum Aktivieren zumindest des Teils der Elektronik zu empfangen, und/oder die ausgebildet ist, ein Signal zum Deaktivieren zumindest des Teils der Elektronik zu empfangen, wobei die Steuerung ausgebildet ist, zumindest den Teil der Elektronik beim Empfang des Signals zum Deaktivieren zu deaktivieren, und/oder wobei die Steuerung dazu ausgebildet ist, zumindest den Teil der Elektronik beim Empfang des Signals zum Aktivieren zu aktivieren.

Es kann vorgesehen sein, dass das Signal zum Aktivieren eine Ortsinformation umfasst, wobei die Steuerung ausgebildet ist, den Teil der Elektronik, der aktiviert wird, abhängig von der Ortsinformation zu aktivieren, und/oder den Teil der Elektronik, der deaktiviert wird, abhängig von der Ortsinformation zu deaktivieren.

Vorzugsweise ist die Steuerung ausgebildet, zumindest den Teil der Elektronik nach einer vorgegebenen Zeit oder einer insbesondere über eine Schnittstelle der Vorrichtung zur Einstellung der Zeit vorgebbaren Zeit nach dem Erkennen des Vorgangs zu aktivieren.

Vorzugsweise ist die Steuerung ausgebildet, zumindest den Teil der Elektronik durch mechanisches oder elektrisches Unterbrechen einer elektrischen Verbindung zwischen der Energiequelle und zumindest dem Teil der Elektronik zu deaktivieren.

Vorzugsweise ist die Steuerung ausgebildet, zumindest den Teil der Elektronik durch Abschalten einer Komponente des Teils der Elektronik zu deaktivieren.

Vorzugsweise ist die Steuerung ausgebildet, zumindest den Teil der Elektronik durch Herstellen einer elektrischen Verbindung zwischen der Energiequelle und zumindest dem Teil der Elektronik zu aktivieren.

Die Steuerung umfasst in einer Ausführungsform ein Formgedächtniselement, insbesondere ein Bi-Metall, welches in Abhängigkeit von der Temperatur mindestens eine erste Form und eine zweite Form annehmen kann, wobei das Formgedächtniselement ausgebildet ist, in einer ersten Form des Formgedächtniselements zumindest den Teil der Elektronik durch Unterbrechen der elektrischen Verbindung zwischen der Energiequelle und zumindest dem Teil der Elektronik zu deaktivieren, und in einer zweiten Form des Formgedächtniselements durch Herstellen der elektrischen Verbindung zwischen der Energiequelle und zumindest den Teil der Elektronik zu aktivieren.

Die Schnittstelle umfasst in einer Ausführungsform eine Spule, insbesondere eine Spule zur Nahfeldkommunikation, vorzugsweise ein NFC-Tag oder RFID-Tag, wobei die Steuerung ausgebildet ist, eine in die Spule induzierte Spannung zu erkennen, und zumindest den Teil der Elektronik bei Erkennen der induzierten Spannung zu aktivieren.

Die Steuerung umfasst in einer Ausführungsform ein Schaltelement, das ausgebildet ist, zumindest den Teil der Elektronik zu aktivieren, wobei das Schaltelement derart an der Vorrichtung angeordnet ist, dass das Schaltelement im Vorgang in Kommunikation oder Kontakt mit einem Sterilgutbehälter für das Sterilgut steht. Das Schaltelement ist in diesem Fall ausgebildet, zumindest den Teil der Elektronik bei Herstellung des Kontakts bzw. der Kommunikation mit dem Sterilgutbehälter zu aktivieren und bei Verlust des Kontakts bzw. der Kommunikation mit dem Sterilgutbehälter zu deaktivieren.

Es kann vorgesehen sein, dass das Schaltelement in einer ersten Stellung des Schaltelements einen Stromfluss eines von der Energiequelle bereitgestellten Stroms durch das Schaltelement ermöglicht, und in einer zweiten Stellung des Schaltelements einen Stromfluss durch das Schaltelement verhindert, wobei die Steuerung ausgebildet ist, die erste Stellung des Schaltelements von der zweiten Stellung des Schaltelements zu unterscheiden, und zumindest den Teil der Elektronik bei Erkennen der ersten Stellung oder eines Wechsels von der zweiten Stellung zur ersten Stellung zu aktivieren.

Bei einem Sterilgutbehälter nach Anspruch 8 ist vorgesehen, dass das Schaltelement in einer ersten Stellung des Schaltelements einen Stromfluss eines von der Energiequelle bereitgestellten Stroms durch das Schaltelement zumindest zum Teil der Elektronik ermöglicht, und in einer zweiten Stellung des Schaltelements einen Stromfluss durch das Schaltelement zumindest zum Teil der Elektronik verhindert, wobei das Schaltelement ausgebildet ist, sich bei Kommunikation oder Kontakt mit dem Sterilgutbehälter in der ersten Stellung zu befinden, und sich bei Verlust der Kommunikation oder des Kontakts mit dem Sterilgutbehälter in die zweite Stellung zu bewegen.

Weiter vorteilhafte Ausführungsformen sind der folgenden Beschreibung und der Zeichnung entnehmbar. In der Zeichnung zeigt:
Fig. 1 eine schematische Darstellung einer Vorrichtung zur Ortung von Sterilgut,
Fig. 2 eine schematische Darstellung eines Vorgangs zum Sterilisieren von Sterilgut,
Fig. 3 ein Druckverlauf und einen Temperaturverlauf bei einem Vorgang zum Sterilisieren von Sterilgut,
Fig. 4 ein Flussdiagramm mit Schritten eines Verfahrens zum Betreiben der Vorrichtung.

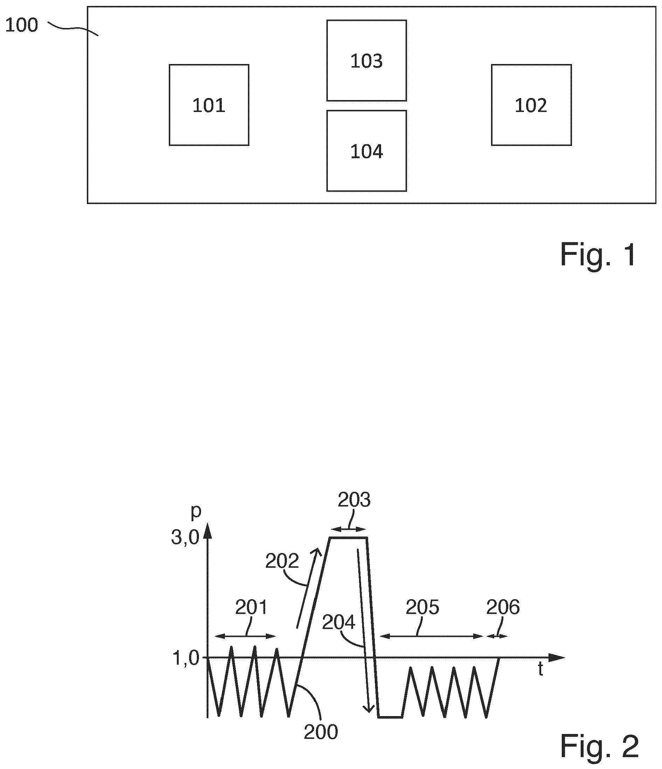

In Figur 1 ist eine Vorrichtung 100 zur Ortung von Sterilgut schematisch dargestellt.

Die Vorrichtung 100 ist dazu ausgebildet, in und/oder an einem Sterilgutbehälter oder in und/oder an einer Einweg-Fliesverpackung für Sterilgut sterilisiert zu werden. Die Vorrichtung 100 kann am Sterilgutbehälter befestigbar oder in den Sterilgutbehälter oder in die Einweg-Fliesverpackung einlegbar ausgeführt sein.

Die Vorrichtung 100 umfasst eine Energiequelle 101, eine Elektronik 102 zum Senden eines Signals zur Ortung durch andere Geräte oder eines Signals zur Ortung, das den Ort des Sterilgutbehälters oder der Einweg-Fliesverpackung umfasst, und eine Steuerung 103. Es kann vorgesehen sein, dass die Vorrichtung 100 eine Schnittstelle 104 umfasst.

Das Signal zur Ortung kann ein Beacon Signal sein, das die Ortung durch die anderen Geräte z.B. mittels Triangulation ermöglicht. Die Vorrichtung 100 kann ausgebildet sein, Signale zur Bestimmung des Orts von anderen Geräten zu empfangen und den Ort abhängig von den Signalen zur Bestimmung des Orts z.B. durch Triangulation zu bestimmen.

Die Energiequelle 101 ist z.B. eine Batterie oder ein Akkumulator.

Die Elektronik 102 umfasst zumindest einen Teil, der deaktivierbar ist.

Die Elektronik 102 kann einen oder mehrere deaktivierbare Teile umfassen. Die Elektronik 102 kann einen nicht deaktivierbaren Teil umfassen.

Zum Schutz der Elektronik 102 im Vorgang ist bei einer Ausführungsform ein Teil der Elektronik 102, der nicht deaktivierbar ist, verkapselt ausgeführt. Der deaktivierbare Teil der Elektronik 102 oder die deaktivierbaren Teile der Elektronik 102 sind offen oder mit im Vorgang durchspülbaren Spalten ausgebildet. Dies bietet große Vorteile gegenüber einer aufwändigen vollständigen Verkapselung der Elektronik 102.

Die Elektronik 102 umfasst z.B. Komponenten wie eine gedruckte Leiterplatte oder einen Sensor, z.B. Temperatursensor, oder einen Microcontroller oder Kondensatoren oder Spulen oder Widerstände oder digitale Signalprozessoren zum Empfangen oder Erzeugen von Signalen.

Alternativ zur Kapselung kann ein Teil der Elektronik auch so ausgeführt sein, dass die Komponenten der Elektronik 102 den während eines Wasch- oder Sterilisationsprozesses auftretenden Belastungen z.B. durch Wasser, Dampf oder Temperatur auch ohne entsprechend zusätzliche Kapselung standhalten.

Die Energiequelle 101 ist in einer weiteren Ausführungsform verkapselt. Dies ermöglicht einen besonders guten Schutz der Energiequelle 101 gegen Kurzschlüsse und/oder zu hohe Temperaturen. Dies ermöglicht auch den Einsatz einer nicht speziell für die hohen Temperaturen und Feuchtigkeit im Vorgang zur Sterilisation ausgelegten Energiequelle 101. Die Energiequelle 101 ist in einer Ausführungsform offen. Dies erfordert den Einsatz einer speziell für die hohen Temperaturen und Feuchtigkeit im Vorgang zur Sterilisation ausgelegten Energiequelle 101 und erspart den Aufwand zur Verkapselung der Energiequelle 101.

Das bedeutet, die Vorrichtung umfasst in einem Beispiel einen verkapselten, stark geschützten und einen weniger stark geschützten z.B. offenen oder mit einem einfacher gestalteten Gehäuse umgebenen Bereich. Der weniger stark geschützte Bereich muss nicht zwingend offen gestaltet sein. Es kann auch ein einfacher gestaltetes Gehäuse sein wie im stark verkapselten Bereich.

Ein Verkapseln der geschützten Bereiche wird z.B. durch Einschluss in ein Gehäuse aus Edelstahl oder aus Edelstahl mit einem Einsatz aus Kunststoff im Bereich der Antenne erreicht. Ein Verkapseln der geschützten Bereiche wird z.B. durch Einschluss in ein Gehäuse aus Kunststoff, welches den alkalischen Reinigungschemikalien widersteht, z.B. PPSU oder PEEK, erreicht. Ein Verkapseln der geschützten Bereiche wird z.B. durch Einschluss in Epoxidharz erreicht. Ein Schutz der geschützten Bereiche insbesondere vor den hohen Prozesstemperaturen wird z.B. durch eine Isolierung, beispielsweise mit PU-Schaum, erreicht.

Die Elektronik 102 ist ausgebildet, ein Signal zur Ortung der Vorrichtung 100 zu erzeugen. Die Elektronik 102 ist ausgebildet, das Signal zu senden.

Die Elektronik 102 umfasst z.B. einen elektrischen Schaltkreis, der ausgebildet ist, das Signal zu erzeugen, und eine Antenne um das Signal zu senden.

Die Energiequelle 101 ist ausgebildet, die Elektronik 102 mit Energie zu versorgen.

Die Steuerung 103 ist ausgebildet, einen Vorgang zum Waschen oder Sterilisieren zu erkennen und zumindest einen Teil der Elektronik 102 im Vorgang zumindest zeitweise zu deaktivieren.

Gemäß einer Ausführungsform ist die Steuerung 103 ausgebildet, eine Temperatur zu erfassen und zumindest den Teil der Elektronik 102 bei einem Erreichen oder Überschreiten eines Schwellwerts zu deaktivieren.

Beispielsweise umfasst die Elektronik 102 einen Temperatursensor, der ausgebildet ist, die Temperatur in der Umgebung der Vorrichtung 100 zu erfassen.

Der Schwellwert kann vorgegeben oder vorgebbar sein und ist bevorzugt ein Temperaturschwellwert.

Alternativ oder zusätzlich umfasst die Elektronik 102 einen Drucksensor, der ausgebildet ist, den Druck in der Umgebung der Vorrichtung 100 zu erfassen und der vorgegebene oder vorgebbare Schwellwert ist ein Druckschwellwert.

Die Steuerung 103 umfasst z.B. einen Speicher, in dem der Temperaturschwellwert gespeichert ist. Die Steuerung 103 umfasst z.B. einen Mikroprozessor, der den Speicher umfasst oder auf den Speicher zugreift und ausgebildet ist, zu erkennen, dass die Temperatur den Temperaturschwellwert erreicht oder überschreitet, und die Elektronik 102 zu deaktivieren, wenn die Temperatur den Temperaturschwellwert erreicht bzw. überschreitet.

Der vorgegebene Temperaturschwellwert ist z.B. in Firmware im Speicher gespeichert. Es kann vorgesehen sein, dass der Temperaturschwellwert über die Schnittstelle 104 vorgebbar ist. Die Schnittstelle 104 umfasst z.B. eine Datenkommunikationsschnittstelle oder ein mechanisches Element, vorzugsweise einen Schalter oder Drehregler, zum Vorgeben des Temperaturschwellwerts.

Es kann vorgesehen sein, dass der Teil der Elektronik 102, der deaktiviert wird, ausgebildet ist, die Temperatur zu erfassen. Dies ist besonders dann besonders vorteilhaft, wenn der deaktivierbare Teil der Elektronik nach einer vorgegebenen oder vorgebbaren Zeit nach dessen Deaktivierung ohne Berücksichtigung eines weiteren Temperaturschwellwerts aktiviert bzw. reaktiviert wird.

In einer Ausführungsform ist die Steuerung 103 ausgebildet, zumindest den Teil der Elektronik 102 erst nach Ablauf einer gewissen Zeit nach dem Erkennen des Vorgangs zum Sterilisieren bzw. nach dem Erreichen des Temperaturschwellwerts zu aktivieren.

Die Zeit kann vorgegeben oder vorgebbar sein.

Die Zeit ist z.B. im Speicher gespeichert. Der Mikroprozessor ist z.B. ausgebildet, den Ablauf der Zeit zu erkennen und die Elektronik 102 zur Prüfung erst zu aktivieren, wenn der Ablauf der Zeit erkannt wird.

Die vorgegebene Zeit ist z.B. in der Firmware im Speicher gespeichert.

Es kann vorgesehen sein, dass die Zeit über die Schnittstelle 104 vorgebbar ist. Beispielsweise ist die Zeit über die Datenkommunikationsschnittstelle oder ein mechanisches Element, vorzugsweise einen Schalter oder Drehregler zur Vorgabe der Zeit, vorgebbar.

Die Steuerung 103 ist z.B. ausgebildet, den deaktivierten Teil der Elektronik 102 in insbesondere regelmäßigen zeitlichen Abständen für eine Prüfung der Temperatur zu aktivieren. In der Prüfung wird z.B. geprüft, ob die Temperatur den Temperaturschwellwert erreicht oder überschreitet oder unterschreitet.

Die Steuerung 103 ist z.B. ausgebildet, abhängig vom Ergebnis der Prüfung zumindest den Teil der Elektronik 102 wieder zu deaktivieren, wenn die Temperatur den Temperaturschwellwert erreicht oder überschreitet.

Die Steuerung 103 ist z.B. ausgebildet, abhängig vom Ergebnis der Prüfung den zuvor deaktivierten Teil der Elektronik 102 aktiviert zu lassen, wenn die Temperatur unterhalb des Temperaturschwellwerts liegt.

Der Mikroprozessor ist z.B. ausgebildet, die Prüfung durchzuführen und zumindest den Teil der Elektronik 102 abhängig vom Ergebnis der Prüfung zu deaktivieren oder aktiviert zu lassen.

Es kann vorgesehen sein, dass die Steuerung 103 ausgebildet ist, zumindest den Teil der Elektronik 102 nach dem Erkennen des Vorgangs zum Sterilisieren zu deaktivieren und den deaktivierten Teil der Elektronik 102 erst nach Ablauf einer Zeitspanne erstmals nach dem Erkennen des Vorgangs zum Sterilisieren zur Prüfung zu aktivieren.

Die Zeitspanne kann vorgegeben oder vorgebbar sein.

Die Zeitspanne ist z.B. im Speicher gespeichert. Der Mikroprozessor ist z.B. ausgebildet ist, den Ablauf der Zeitspanne zu erkennen und die Elektronik 102 zur Prüfung erstmals zu aktivieren, wenn der Ablauf der Zeitspanne erkannt wird.

Die vorgegebene Zeitspanne ist z.B. in der Firmware im Speicher gespeichert.

Es kann vorgesehen sein, dass der Temperaturschwellwert über die Schnittstelle 104 vorgebbar ist. Die Schnittstelle 104 umfasst z.B. eine Datenkommunikationsschnittstelle oder ein mechanisches Element, vorzugsweise einen Schalter oder Drehregler, zum Vorgeben der Zeitspanne.

In einer Ausführungsform ist die Schnittstelle 104 zur Signalkommunikation ausgebildet.

Die Schnittstelle 104 ist z.B. ausgebildet, ein Signal zum Deaktivieren zumindest des Teils der Elektronik 102 zu empfangen, wobei die Steuerung 103 ausgebildet, zumindest den Teil der Elektronik 102 beim Empfang des Signals zum Deaktivieren zu deaktivieren.

Die Schnittstelle 104 ist z.B. ausgebildet, ein Signal zum Aktivieren zumindest des Teils der Elektronik 102 zu empfangen, wobei die Steuerung 103 dazu ausgebildet ist, zumindest den Teil der Elektronik 102 beim Empfang des Signals zum Aktivieren zu aktivieren.

In einer Ausführungsform ist die Steuerung 103 ausgebildet, den Teil der Elektronik 102, der aktiviert oder deaktiviert wird, abhängig von einer Ortsinformation zu aktivieren oder zu deaktivieren.

Beispielsweise ist vorgesehen, das Signal zum Deaktivieren oder das Signal zum Aktivieren abhängig von Ortsinformation zu senden. Die Ortsinformation ist im Beispiel eine Angabe über einen Raum, in dem sich die Vorrichtung 100 befindet. Beispielsweise wird durch die Ortsinformation eine unerwünschte Deaktivierung vermieden.

In einer Ausführungsform ist die Steuerung 103 ausgebildet, den Ort der Vorrichtung 100 zur Vermeidung einer unerwünschten Deaktivierung zu bestimmen. Beispielsweise wird durch die Auswertung des Ortes eine unerwünschte Deaktivierung wegen einer Temperatur über dem Temperaturschwellwert vermieden, die ansonsten durch Sonneneinwirkung bei einem Transport oder Lichtquellen in einem Operationssaal entsteht.

In einer Ausführungsform ist die Steuerung 103 ausgebildet, zumindest den Teil der Elektronik 102 durch mechanisches oder elektrisches Unterbrechen einer elektrischen Verbindung zwischen der Energiequelle 101 und zumindest dem Teil der Elektronik 102 zu deaktivieren.

Die Trennung zumindest des Teils der Elektronik 102 von der Energiequelle 101 bietet diverse Vorteile, insbesondere für die Nutzung im Bereich der Steriltechnik. Dadurch, dass die Elektronik 102 stromlos oder teilweise stromlos ist, werden Kurzschlüsse innerhalb der Elektronik 102 vermieden. Dies ist insbesondere für die feuchtigkeitsintensiven maschinellen Aufbereitungsprozesse der Sterilgutaufbereitung vorteilhaft.

In einer Ausführungsform ist die Steuerung 103 ausgebildet, zumindest den Teil der Elektronik 102 durch Abschalten einer Komponente des Teils der Elektronik 102 zu deaktivieren.

In einer Ausführungsform ist die Steuerung 103 ausgebildet, zumindest den Teil der Elektronik 102 durch Herstellen einer elektrischen Verbindung zwischen der Energiequelle 101 und zumindest dem Teil der Elektronik 102 zu aktivieren.

Die Vorrichtung 100 oder die Elektronik 102 umfasst z.B. einen Schalter zum Herstellen oder Unterbrechen der elektrischen Verbindung.

In einer Ausführungsform umfasst die Steuerung 103 ein Formgedächtniselement. Das Formgedächtniselement ist z.B. ein Bi-Metall. Das Formgedächtniselement ist z.B. ausgebildet, in einer ersten Form des Formgedächtniselements zumindest den Teil der Elektronik 102 durch Unterbrechen der elektrischen Verbindung zwischen der Energiequelle 101 und zumindest dem Teil der Elektronik 102 zu deaktivieren.

Das Formgedächtniselement ist z.B. ausgebildet, in einer zweiten Form des Formgedächtniselements zumindest den Teil der Elektronik 102 durch Herstellen der elektrischen Verbindung zwischen der Energiequelle 101 und zumindest dem Teil der Elektronik 102 zu aktivieren.

Das Formgedächtniselement ist z.B. als Schalter zum Herstellen oder Unterbrechen der elektrischen Verbindung ausgebildet oder ausgebildet, den Schalter zum Herstellen oder Unterbrechen der elektrischen Verbindung zu schalten.

In einer Ausführungsform umfasst die Schnittstelle 104 eine Spule. Die Steuerung 103 ist ausgebildet, eine in der Spule induzierte Spannung zu erkennen und zumindest den Teil der Elektronik 102 bei Erkennen der induzierten Spannung zu aktivieren. Die Spule ist z.B. eine Spule zur Nahfeldkommunikation, vorzugsweise ein NFC-Tag oder RFID-Tag.

In einer Ausführungsform ist die Steuerung 103 ausgebildet, den Schalter zum Herstellen oder Unterbrechen der elektrischen Verbindung abhängig von der induzierten Spannung zu schalten.

In einer Ausführungsform umfasst die Steuerung 103 ein Schaltelement. Das Schaltelement ist ausgebildet, zumindest den Teil der Elektronik 102 zu aktivieren.

Das Schaltelement ist derart an der Vorrichtung 100 angeordnet, dass das Schaltelement im Vorgang in Kommunikation oder Kontakt mit einem Sterilgutbehälter für das Sterilgut steht.

Das Schaltelement ist ausgebildet, zumindest den Teil der Elektronik 102 bei Verlust des Kontakts mit dem Sterilgutbehälter zu deaktivieren.

In einer Ausführungsform ermöglicht das Schaltelement in einer ersten Stellung des Schaltelements einen Stromfluss eines von der Energiequelle 101 bereitgestellten Stroms durch das Schaltelement und verhindert in einer zweiten Stellung des Schaltelements einen Stromfluss durch das Schaltelement.

In einer Ausführungsform ist die Steuerung 103 ausgebildet, die erste Stellung des Schaltelements von der zweiten Stellung des Schaltelements zu unterscheiden und zumindest den Teil der Elektronik 102 bei Erkennen der ersten Stellung oder eines Wechsels von der zweiten Stellung zur ersten Stellung zu aktivieren.

In einer Ausführungsform ist das Schaltelement ausgebildet, sich bei Kontakt mit dem Sterilgutbehälter in der zweiten Stellung zu befinden und sich bei Verlust des Kontakts mit dem Sterilgutbehälter in die erste Stellung zu bewegen.

Figur 2 stellt einen Verlauf 200 eines Drucks p über der Zeit t in einem Vorgang zum Waschen oder Sterilisieren von Sterilgut am Beispiel einer beispielhaften Dampfsterilisation schematisch dar.

Der Druck p bewegt sich im Beispiel unterhalb von 1 bar und oberhalb von 1 bar bis 3.1 bar in unterschiedlichen Phasen:
201 fraktioniertes Vakuum
202 Steigezeit
203 Sterilisationszeit
204 Druckabfall
205 fraktionierte Trocknung
206 Belüftung

Die Phasen fraktioniertes Vakuum 201 und Steigezeit 202 dauern z.B. bis kontaminierte Luft entfernt bzw. die Temperatur für die Sterilisationszeit 203 erreicht ist. Die Sterilisationszeit 203 beträgt z.B. mindestens 3 Minuten bei einer Temperatur von 134°C. Die fraktionierte Trocknung 205 und Belüftung 206 sind Teile einer Trocknungszeit.

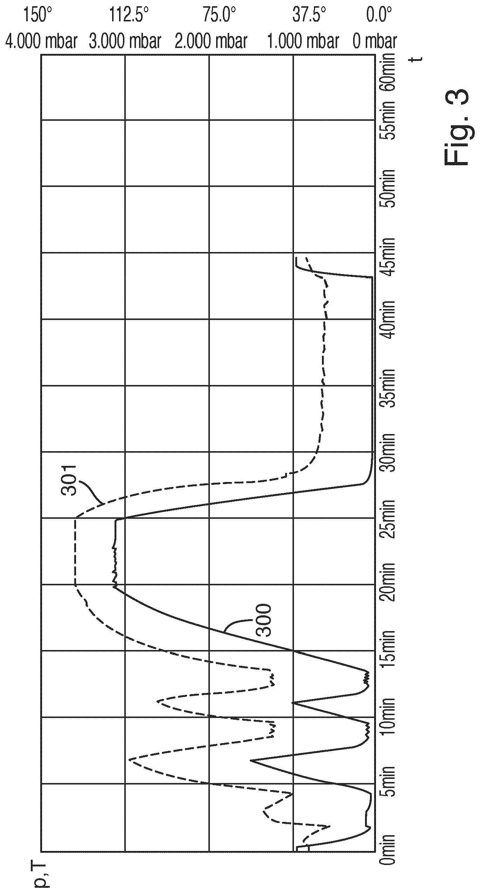

Figur 3 stellt einen Verlauf 300 eines Drucks p zwischen 0 Millibar und ~3150 Millibar (0 bis 3,15 * 10^5 Pa) sowie einen Verlauf 301 einer Temperatur T zwischen Zimmertemperatur und 135° Celsius für 45 Minuten eines Vorgangs zum Sterilisieren von Sterilgut am Beispiel einer Dampfsterilisation mit fraktioniertem Vakuum und 15 Minuten Trocknungszeit am Ende des Vorgangs dar.

Für die Energiequelle 101 sind hohe Temperaturen und insbesondere die im Prozess maximal erreichte und gehaltene Temperatur von 135° Celsius kritisch. Zum Schutz der Energiequelle 101 ist im Beispiel ein Deaktivieren zumindest des Teils der Elektronik 102 zumindest in dem Bereich des Vorgangs, in dem die Temperatur 135° Celsius oder im Wesentlichen 135° Celsius beträgt, vorgesehen. Das bedeutet, der Temperaturschwellwert für den beispielhaften Verlauf wird kleiner als 135° Celsius, vorteilhafterweise zwischen 70° Celsius und 80° Celsius, gewählt.

Sofern für die Elektronik 102 Feuchtigkeit, die im Vorgang entsteht, kritisch ist, kann diese in der Trocknungszeit reduziert werden.

In einem Beispiel wird die Zeit, die bis zur Aktivierung der Elektronik 102 abgewartet wird, so gewählt, dass die Aktivierung am Ende der Trocknungszeit erfolgt. Das bedeutet, es wird im beispielhaften Verlauf ungefähr die Sterilisationszeit 203, die Zeit des Druckabfalls 204 und die Trocknungszeit, im beispielhaften Verlauf 30 Minuten nach Erkennen des Vorgangs, gewartet.

Besonders Vorteilhaft kann die Aktivierung nach einer Abkühlungszeit, beispielsweise von 30 Minuten nach Ende der Trocknungszeit, erfolgen.

Für unterschiedliche Sterilgüter oder Autoklaven oder Krankenhäuser können unterschiedliche Verläufe zur Sterilisierung mit unterschiedlichen Temperaturen oder Drücken oder Zeitdauern der Phasen oder eine andere Zusammensetzung der Phasen vorgesehen sein. Der Temperaturschwellwert und die Zeit, die vor der Aktivierung gewartet wird, sind entsprechend angepasst.

Für das Aktivieren und Deaktivieren können unterschiedliche Temperaturschwellwerte für die Temperatur vorgesehen sein. Beispielsweise ist ein niedrigerer Temperaturschwellwert für die Aktivierung vorgesehen als für die Deaktivierung.

In einem Beispiel, in dem die Steuerung 103 das Formgedächtniselement umfasst, wird zumindest der Teil der Elektronik 102 durch Herstellen der elektrischen Verbindung zwischen der Energiequelle 101 und zumindest dem Teil der Elektronik 102 aktiviert. In einem Beispiel, in dem die Steuerung 103 das Formgedächtniselement umfasst, wird zumindest der Teil der Elektronik 102 durch Unterbrechen der elektrischen Verbindung zwischen der Energiequelle 101 und zumindest dem Teil der Elektronik 102 deaktiviert.

In einem Beispiel, in dem die Steuerung 103 das Schaltelement umfasst, wird zumindest der Teil der Elektronik 102 bei Kontakt zwischen dem Schaltelement und dem Sterilgutbehälter aktiviert. Beispielsweise bewegt sich das Schaltelement beim Kontakt in die erste Stellung wodurch die elektrische Verbindung zwischen der Energiequelle 101 und zumindest dem Teil der Elektronik 102 hergestellt **wird.**

In einem Beispiel, in dem die Steuerung 103 das Schaltelement umfasst, wird zumindest der Teil der Elektronik 102 bei Verlust des Kontakts zwischen dem Schaltelement und dem Sterilgutbehälter deaktiviert. Beispielsweise bewegt sich das Schaltelement beim Verlust des Kontakts in die zweite Stellung, wodurch die elektrische Verbindung zwischen der Energiequelle 101 und zumindest dem Teil der Elektronik 102 unterbrochen bzw. getrennt wird.

Die Steuerung 103 ist in einem Beispiel ausgebildet, ein Verfahren zum Betreiben der Vorrichtung 100 auszuführen.

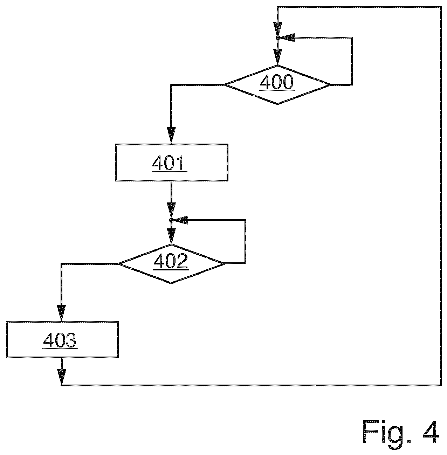

In Figur 4 ist ein Flussdiagramm mit Schritten des Verfahrens zum Betreiben der Vorrichtung 100 dargestellt.

Die Vorrichtung 100 ist im Beispiel zu Beginn des Verfahrens in einem Zustand, in dem die gesamte Elektronik 102 aktiviert ist.

Das Verfahren umfasst einen Schritt 400.

Im Schritt 400 wird geprüft, ob ein Vorgang zum Waschen oder Sterilisieren erkannt wird oder nicht.

Wenn der Vorgang erkannt wird, wird ein Schritt 401 ausgeführt. Anderenfalls wird der Schritt 400 ausgeführt. Vorzugsweise erfolgt wie wiederholte Ausführung des Schritts 400 mit einer gewissen Verzögerung.

Der Vorgang wird abhängig von der erfassten Temperatur erkannt.

Beispielsweise wird die Temperatur erfasst und festgestellt, dass zumindest der Teil der Elektronik 102 zu deaktivieren ist, wenn die Temperatur den Temperaturschwellwert erreicht oder überschreitet oder überschritten hat.

Es kann vorgesehen sein, dass im Schritt 400 das Signal zum Deaktivieren zumindest des Teils der Elektronik 102 empfangen wird, und beim Empfang des Signals zum Deaktivieren festgestellt wird, dass zumindest der Teil der Elektronik 102 zu deaktivieren ist.

In einem Beispiel, in dem die Steuerung 103 das Schaltelement umfasst, wird zumindest der Teil der Elektronik 102 bei Kontakt zwischen dem Schaltelement und dem Sterilgutbehälter aktiviert. Beispielsweise bewegt sich das Schaltelement beim Kontakt in die erste Stellung, wodurch der Kontakt von der Steuerung 103 erkannt wird und die Steuerung 103 zumindest den Teil der Elektronik 102 aktiviert.

Im Schritt 401 wird zumindest der Teil der Elektronik 102 im Vorgang deaktiviert.

Der Teil der Elektronik 102 wird z.B. beim Erkennen des Vorgangs zum Sterilisieren oder nach Ablauf der Zeit nach dem Erkennen des Vorgangs zum Sterilisieren aktiviert.

Es kann vorgesehen sein, dass das Signal zum Deaktivieren die Ortsinformation umfasst, wobei der Teil der Elektronik 102, der deaktiviert wird, abhängig von der Ortsinformation deaktiviert wird.

In einem Beispiel wird zumindest der Teil der Elektronik 102 durch Abschalten einer Komponente der Elektronik 102 deaktiviert.

In einem Beispiel wird zumindest der Teil der Elektronik 102 durch mechanisches oder elektrisches Unterbrechen der elektrischen Verbindung zwischen der Energiequelle 101 und zumindest dem Teil der Elektronik 102 deaktiviert.

Anschließend wird ein Schritt 402 ausgeführt.

Im Schritt 402 wird geprüft, ob zumindest der Teil der Elektronik 102 zu aktivieren ist oder nicht.

Wenn zumindest der Teil der Elektronik 102 zu aktivieren ist, wird ein Schritt 403 ausgeführt. Anderenfalls wird der Schritt 402 ausgeführt.

Ein anderer als der deaktivierte Teil der Elektronik 102 ist zum Erfassen der Temperatur ausgebildet, wobei festgestellt wird, dass der deaktivierte Teil der Elektronik 102 zu aktivieren ist, wenn festgestellt wird, dass die Temperatur den Temperaturschwellwert unterschreitet.

Es kann vorgesehen sein, dass im Schritt 402 das Signal zum Aktivieren zumindest des Teils der Elektronik 102 empfangen wird und beim Empfang des Signals zum Aktivieren festgestellt wird, dass zumindest der Teil der Elektronik 102 zu aktivieren ist.

Die Trocknungsvorgänge der Reinigung und Desinfektion und des Vorgangs zur Sterilisierung werden durch entsprechende Ausgestaltung des Temperaturschwellwerts für die Temperatur, der Zeitspanne und/oder eines Zeitpunkts, zu dem das Signal zum Aktivieren gesendet wird, genutzt, um potentielle Restfeuchtigkeit in der Elektronik 102 vor dem Aktivieren zu eliminieren.

Im Schritt 403 wird zumindest der Teil der Elektronik 102 aktiviert.

In einem Beispiel wird zumindest der Teil der Elektronik 102 durch Einschalten der Komponente der Elektronik 102 aktiviert.

In einem Beispiel wird zumindest der Teil der Elektronik 102 durch mechanisches oder elektrisches Herstellen der elektrischen Verbindung zwischen der Energiequelle 101 und zumindest dem Teil der Elektronik 102 aktiviert.

In einem Beispiel, in dem die Schnittstelle 104 die Spule umfasst, wird die in der Spule induzierte Spannung erkannt, und zumindest der Teil der Elektronik 102 bei Erkennen der induzierten Spannung und/oder durch die induzierte Spannung aktiviert.

In einem Beispiel, in dem die Steuerung 103 das Schaltelement umfasst, wird zumindest der Teil der Elektronik 102 bei Verlust des Kontakts zwischen dem Schaltelement und dem Sterilgutbehälter aktiviert. Beispielsweise bewegt sich das Schaltelement beim Verlust in die erste Stellung, wodurch der Verlust des Kontakts von der Steuerung 103 erkannt und zumindest der Teil der Elektronik 102 durch die Steuerung 103 aktiviert wird.

Es kann vorgesehen sein, dass das Signal zum Aktivieren abhängig von der Ortsinformation erfolgt.

Anschließend wird im Beispiel der Schritt 400 ausgeführt. Es kann vorgesehen sein, dass das Verfahren nach dem Schritt 403 endet.

Das Verfahren kann vorsehen, dass der Temperaturschwellwert für die Temperatur und/oder die Zeit, bis zu deren Ablauf vor dem Deaktivieren zumindest des Teils der Elektronik gewartet wird, und/oder die Zeitspanne über die Schnittstelle 104, d.h. z.B. über die Datenkommunikationsschnittstelle oder das mechanisches Element, d.h. den Schalter oder Drehregler, vorgegeben wird.

## Patentansprüche

1. Sterilisierbare Vorrichtung (100) zur Ortung von Sterilgut, die eine Energiequelle (101) und eine Elektronik (102) zum Senden eines Signals zur Ortung umfasst, wobei die Energiequelle (101) ausgebildet ist, die Elektronik (101) mit Energie zu versorgen, wobei die Vorrichtung (100) eine Steuerung (103) umfasst, die ausgebildet ist, einen Vorgang zum Waschen oder Sterilisieren zu erkennen, und einen oder mehrere Teile der Elektronik (102) im Vorgang zumindest zeitweise zu deaktivieren, **dadurch gekennzeichnet, dass** die Elektronik (102) den oder die deaktivierbaren Teile der Elektronik (102) und einen nicht deaktivierbaren Teil umfasst, wobei
- der oder die deaktivierbaren Teile der Elektronik (102) offen und/oder im Vorgang durchspülbar ausgebildet ist, und der nicht deaktivierbare Teil der Elektronik (102) verkapselt ist,
oder
- wobei die Elektronik (102) offen und/oder im Vorgang durchspülbar ist und die Energiequelle (101) verkapselt ist.

2. Vorrichtung (100) nach Anspruch 1, **dadurch gekennzeichnet, dass** die Steuerung (103) ausgebildet ist, eine Temperatur zu erfassen, und zumindest den oder die deaktivierbaren Teile der Elektronik (102) bei einem Erreichen oder Überschreiten eines vorgegebenen Temperaturschwellwerts oder eines insbesondere über eine Schnittstelle (104) der Vorrichtung (100) zur Einstellung der Temperatur vorgebbaren Temperaturschwellwerts zu deaktivieren.

3. Vorrichtung (100) nach Anspruch 2, **dadurch gekennzeichnet, dass** die Steuerung (103) ausgebildet ist, zumindest den oder die deaktivierbaren Teile der Elektronik (102) bei einem Erreichen oder Unterschreiten eines vorgegebenen Temperaturschwellwerts oder eines insbesondere über eine Schnittstelle (104) der Vorrichtung (100) zur Einstellung der Temperatur vorgebbaren Temperaturschwellwerts zu aktivieren.

4. Vorrichtung (100) nach einem der vorherigen Ansprüche, **dadurch gekennzeichnet, dass** die Vorrichtung (100) eine Schnittstelle (104) zur Signalkommunikation umfasst, die ausgebildet ist, ein Signal zum Aktivieren zumindest des oder der deaktivierbaren Teile der Elektronik (102) zu empfangen und/oder die ausgebildet ist, ein Signal zum Deaktivieren zumindest des oder der deaktivierbaren Teile der Elektronik (102) zu empfangen, wobei die Steuerung (103) ausgebildet ist, zumindest den oder die deaktivierbaren Teile der Elektronik (102) beim Empfang des Signals zum Deaktivieren zu deaktivieren, und/oder wobei die Steuerung (103) dazu ausgebildet ist, zumindest den oder die deaktivierbaren Teile der Elektronik (102) beim Empfang des Signals zum Aktivieren zu aktivieren.

5. Vorrichtung (100) nach einem der vorherigen Ansprüche, **dadurch gekennzeichnet, dass** die Steuerung (103) ausgebildet ist, zumindest den oder die deaktivierbaren Teile der Elektronik (102) nach einer vorgegebenen Zeit oder einer insbesondere über eine Schnittstelle (104) der Vorrichtung zur Einstellung der Zeit vorgebbaren Zeit nach dem Erkennen des Vorgangs zu aktivieren.

6. Vorrichtung (100) nach einem der vorherigen Ansprüche, **dadurch gekennzeichnet, dass** die Steuerung (103) ein Formgedächtniselement, insbesondere ein Bi-Metall, umfasst, welches in Abhängigkeit von der Temperatur mindestens eine erste Form und eine zweite Form annehmen kann, wobei das Formgedächtniselement ausgebildet ist, in einer ersten Form des Formgedächtniselements zumindest den oder die deaktivierbaren Teile der Elektronik (102) durch Unterbrechen der elektrischen Verbindung zwischen der Energiequelle (101) und zumindest dem oder den deaktivierbaren Teilen der Elektronik (102) zu deaktivieren, und in einer zweiten Form des Formgedächtniselements durch Herstellen der elektrischen Verbindung zwischen der Energiequelle (101) und zumindest den oder die deaktivierbaren Teile der Elektronik (102) zu aktivieren.

7. Vorrichtung (100) nach einem der vorherigen Ansprüche, **dadurch gekennzeichnet, dass** die Schnittstelle (104) eine Spule, insbesondere eine Spule zur Nahfeldkommunikation, vorzugsweise ein NFC-Tag oder RFID-Tag, umfasst, wobei die Steuerung (103) ausgebildet ist, eine in die Spule induzierte Spannung zu erkennen, und zumindest den oder die deaktivierbaren Teile der Elektronik (102) bei Erkennen der induzierten Spannung zu aktivieren.

8. Sterilgutbehälter für das Sterilgut, mit der Vorrichtung (100) nach einem der vorherigen Ansprüche, **dadurch gekennzeichnet, dass** die Steuerung (103) ein Schaltelement umfasst, das ausgebildet ist, zumindest den oder die deaktivierbaren Teile der Elektronik (102) zu aktivieren, wobei das Schaltelement derart an der Vorrichtung angeordnet ist, dass das Schaltelement im Vorgang in Kommunikation oder Kontakt mit dem Sterilgutbehälter für das Sterilgut steht, wobei das Schaltelement ausgebildet ist, zumindest den oder die deaktivierbaren Teile der Elektronik (102) bei Verlust des Kontakts mit dem Sterilgutbehälter zu aktivieren.

9. Verfahren zum Betreiben einer Vorrichtung zur Ortung von Sterilgut, die eine Energiequelle (101) und eine Elektronik (102) umfasst, wobei die Energiequelle (101) ausgebildet ist, die Elektronik (101) mit Energie zu versorgen, wobei im Verfahren ein Vorgang zum Waschen oder Sterilisieren erkannt (400) und zumindest ein oder mehrere deaktivierbare Teile der Elektronik (102) im Vorgang zumindest zeitweise deaktiviert werden (401), **dadurch gekennzeichnet, dass** der oder die deaktivierten Teile der Elektronik (102) im Vorgang durchgespült werden und dass die Energiequelle (101) verkapselt wird.

10. Verfahren nach Anspruch 9, **dadurch gekennzeichnet, dass** eine Temperatur erfasst wird und zumindest der oder die deaktivierbaren Teile der Elektronik (102) bei einem Erreichen oder Überschreiten eines Temperaturschwellwerts deaktiviert werden.

11. Verfahren nach Anspruch 10, **dadurch gekennzeichnet, dass** der oder die deaktivierten deaktivierbaren Teile der Elektronik (102) in insbesondere regelmäßigen zeitlichen Abständen aktiviert werden, geprüft wird, ob die Temperatur den Temperaturschwellwert unterschreitet, und der oder die deaktivierbaren Teile der Elektronik (102) deaktiviert werden, wenn die Temperatur den Temperaturschwellwert erreicht oder überschreitet, oder wobei der oder die aktivierten deaktivierbaren Teile der Elektronik (102) anderenfalls aktiviert gelassen werden.

12. Verfahren nach einem der Ansprüche 9 bis 11, **dadurch gekennzeichnet, dass** ein Signal zum Aktivieren zumindest des oder der deaktivierbaren Teile der Elektronik (102) empfangen wird, wobei zumindest der oder die deaktivierbaren Teile der Elektronik (102) beim Empfang des Signals zum Aktivieren aktiviert werden, und/oder wobei ein Signal zum Deaktivieren zumindest des oder der deaktivierbaren Teile der Elektronik (102) empfangen wird, wobei zumindest der oder die deaktivierbaren Teile der Elektronik (102) beim Empfang des Signals zum Deaktivieren deaktiviert werden.

13. Verfahren nach einem der Ansprüche 9 bis 12, **dadurch gekennzeichnet, dass** zumindest der oder die deaktivierbaren Teile der Elektronik (102) erst nach einer Zeit nach dem Erkennen des Vorgangs zum Sterilisieren aktiviert werden.

14. Verfahren nach einem der Ansprüche 9 bis 13, **dadurch gekennzeichnet, dass** die Vorrichtung (100) eine Spule, insbesondere eine Spule zur Nahfeldkommunikation, vorzugsweise ein NFC-Tag oder RFID-Tag, umfasst, wobei eine in die Spule induzierte Spannung erkannt und zumindest der oder die deaktivierbaren Teile der Elektronik (102) bei Erkennen der induzierten Spannung aktiviert werden.

## Claims

1. Sterilizable device (100) for locating sterile goods, said device comprising a power source (101) and electronics (102) for transmitting a locating signal, wherein the power source (101) is designed to supply the electronics (101) with power, wherein the device (100) comprises a controller (103) designed to detect a washing or sterilization process and to at least temporarily deactivate one or more parts of the electronics (102) during the process, **characterized in that** the electronics (102) comprise the deactivatable part or parts of the electronics (102) and a non-deactivatable part, wherein
- the deactivatable part or parts of the electronics (102) are open and/or rinsable during the process, and the non-deactivatable part of the electronics (102) is encapsulated,
or
- wherein the electronics (102) are open and/or rinsable during the process and the power source (101) is encapsulated.

2. Device (100) according to claim 1, **characterized in that** the controller (103) is designed to record a temperature and to deactivate at least the deactivatable part or parts of the electronics (102) when a specified temperature threshold or a temperature threshold specifiable in particular via an interface (104) of the device (100) for setting the temperature is reached or exceeded.

3. Device (100) according to claim 2, **characterized in that** the controller (103) is designed to activate at least the deactivatable part or parts of the electronics (102) when a specified temperature threshold or a temperature threshold specifiable in particular via an interface (104) of the device (100) for setting the temperature is reached or fallen below.

4. Device (100) according to any of the preceding claims,
**characterized in that** the device (100) comprises a signal communication interface (104) designed to receive a signal to activate at least the deactivatable part or parts of the electronics (102) and/or designed to receive a signal to deactivate at least the deactivatable part or parts of the electronics (102), wherein the controller (103) is designed to deactivate at least the deactivatable part or parts of the electronics (102) when the deactivation signal is received, and/or wherein the controller (103) is designed to activate at least the deactivatable part or parts of the electronics (102) when the activation signal is received.

5. Device (100) according to any of the preceding claims,
**characterized in that** the controller (103) is designed to activate at least the deactivatable part or parts of the electronics (102) after a specified time or a time specifiable in particular via an interface (104) of the device for setting the time, after the process has been detected.

6. Device (100) according to any of the preceding claims, **characterized in that** the controller (103) comprises a shape memory element, in particular a bimetal, which can assume at least a first form and a second form depending on the temperature, wherein the shape memory element is designed to deactivate at least the deactivatable part or parts of the electronics (102) in a first shape of the shape memory element by interrupting the electrical connection between the power source (101) and at least the deactivatable part or parts of the electronics (102), and to activate at least said deactivatable part or parts in a second shape of the shape memory element by establishing the electrical connection between the power source (101) and at least the deactivatable part or parts of the electronics (102).

7. Device (100) according to any of the preceding claims, **characterized in that** the interface (104) comprises a coil, in particular a coil for near field communication, preferably an NFC tag or an RFID tag, wherein the controller (103) is designed to detect a voltage induced in the coil and to activate at least the deactivatable part or parts of the electronics (102) when the induced voltage is detected.

8. Sterile goods container for the sterile goods, comprising the device (100) according to any of the preceding claims, **characterized in that** the controller (103) comprises a switching element designed to activate at least the deactivatable part or parts of the electronics (102), wherein the switching element is arranged on the device in such a way that the switching element is in communication or contact with the sterile goods container for the sterile goods during the process, wherein the switching element is designed to activate at least the deactivatable part or parts of the electronics (102) when contact is lost with the sterile goods container.

9. Method for operating a device for locating sterile goods, said device comprising a power source (101) and electronics (102), wherein the power source (101) is designed to supply the electronics (101) with power, wherein in the method a washing or sterilization process is detected (400) and at least one or more deactivatable parts of the electronics (102) are at least temporarily deactivated (401) during the process, **characterized in that** the deactivated part or parts of the electronics (102) are rinsed during the process and **in that** the power source (101) is encapsulated.

10. Method according to claim 9, **characterized in that** a temperature is recorded and at least the deactivatable part or parts of the electronics (102) are deactivated when a temperature threshold is reached or exceeded.

11. Method according to claim 10, **characterized in that** the deactivated deactivatable part or parts of the electronics (102) are activated at in particular regular time intervals, there is a check as to whether the temperature has fallen below the temperature threshold, and the deactivatable part or parts of the electronics (102) are deactivated if the temperature reaches or exceeds the temperature threshold, or wherein the activated deactivatable part or parts of the electronics (102) are otherwise left activated.

12. Method according to any of claims 9 to 11, **characterized in that** a signal to activate at least the deactivatable part or parts of the electronics (102) is received, wherein at least the deactivatable part or parts of the electronics (102) are activated the activation signal is received, and/or wherein a signal to deactivate at least the deactivatable part or parts of the electronics (102) is received, wherein at least the deactivatable part or parts of the electronics (102) are deactivated when the deactivation signal is received.

13. Method according to any of claims 9 to 12, **characterized in that** at least the deactivatable part or parts of the electronics (102) are activated only after a time after the sterilization process has been detected.

14. Method according to any of claims 9 to 13, **characterized in that** the device (100) comprises a coil, in particular a coil for near field communication, preferably an NFC tag or an RFID tag, wherein a voltage induced in the coil is detected and at least the deactivatable part or parts of the electronics (102) are activated when the induced voltage is detected.

## Revendications

1. Dispositif (100) stérilisable pour la localisation de matériel stérile, comprenant une source d'énergie (101) et une électronique (102) pour l'envoi d'un signal de localisation, la source d'énergie (101) étant conçue pour alimenter en énergie l'électronique (102), le dispositif (100) comprenant une unité de commande (103) conçue pour détecter un procédé de lavage ou de stérilisation, et pour désactiver au moins une ou plusieurs parties désactivables de l'électronique (102) au cours du procédé, **caractérisé en ce que** l'électronique (102) comprend les parties désactivables de l'électronique (102) et une partie non désactivable, où
- les parties désactivables de l'électronique (102) sont conçues ouvertes et/ou rinçables pendant le procédé, et la partie non désactivable de l'électronique (102) est encapsulée,
ou
- l'électronique (102) étant ouverte et/ou rinçable pendant le procédé et la source d'énergie (101) étant encapsulée.

2. Dispositif (100) selon la revendication 1, **caractérisé en ce que** l'unité de commande (103) est conçue pour détecter une température, et pour désactiver au moins une ou plusieurs parties désactivables de l'électronique (102) lors de l'atteinte ou du dépassement d'un seuil de température prédéterminé ou d'un seuil de température pouvant être notamment fixé via une interface (104) du dispositif (100) pour le réglage de la température.

3. Dispositif (100) selon la revendication 2, **caractérisé en ce que** l'unité de commande (103) est conçue pour activer au moins une ou plusieurs parties désactivables de l'électronique (102) lors de l'atteinte ou du franchissement à la baisse d'un seuil de température prédéterminé ou d'un seuil de température pouvant être notamment défini via une interface (104) du dispositif (100) pour le réglage de la température.

4. Dispositif (100) selon l'une quelconque des revendications précédentes, **caractérisé en ce que** le dispositif (100) comprend une interface (104) pour la communication de signaux, conçue pour recevoir un signal d'activation visant au moins une ou plusieurs parties désactivables de l'électronique (102) et/ou conçue pour recevoir un signal de désactivation visant au moins une ou plusieurs parties désactivables de l'électronique (102), l'unité de commande (103) étant conçue pour désactiver au moins une ou plusieurs parties désactivables de l'électronique (102) lors de la réception du signal de désactivation, et/ou l'unité de commande (103) étant conçue pour activer au moins une ou plusieurs parties désactivables de l'électronique (102) lors de la réception du signal d'activation.

5. Dispositif (100) selon l'une quelconque des revendications précédentes, **caractérisé en ce que** l'unité de commande (103) est conçue pour activer au moins une ou plusieurs parties désactivables de l'électronique (102) après un temps prédéterminé ou après un temps pouvant être fixé, notamment via une interface (104) du dispositif, après la détection du procédé.

6. Dispositif (100) selon l'une quelconque des revendications précédentes, **caractérisé en ce que** l'unité de commande (103) comprend un élément à mémoire de forme, notamment un bimétal, pouvant prendre en fonction de la température au moins une première forme et une seconde forme, l'élément à mémoire de forme étant conçu, dans une première forme de l'élément à mémoire de forme, pour désactiver au moins une ou plusieurs parties désactivables de l'électronique (102) en interrompant la connexion électrique entre la source d'énergie (101) et au moins ladite ou lesdites parties désactivables de l'électronique (102), et, dans une seconde forme de l'élément à mémoire de forme, pour activer en rétablissant la connexion électrique entre la source d'énergie (101) et au moins ladite ou lesdites parties désactivables de l'électronique (102).

7. Dispositif (100) selon l'une quelconque des revendications précédentes, **caractérisé en ce que** l'interface (104) comprend une bobine, en particulier une bobine pour la communication en champ proche, de préférence une étiquette NFC ou une étiquette RFID, l'unité de commande (103) étant conçue pour détecter une tension induite dans la bobine, et pour activer au moins une ou plusieurs parties désactivables de l'électronique (102) lors de la détection de la tension induite.

8. Conteneur pour le matériel stérile, comprenant le dispositif (100) selon l'une quelconque des revendications précédentes, **caractérisé en ce que** l'unité de commande (103) comprend un élément de commutation conçu pour activer au moins la ou les parties désactivables de l'électronique (102), l'élément de commutation étant disposé sur le dispositif de telle sorte que, lors de l'opération, il est en communication ou en contact avec le conteneur pour le matériel stérile, l'élément de commutation étant conçu pour activer au moins la ou les parties désactivables de l'électronique (102) en cas de perte du contact avec le conteneur pour le matériel stérile.

9. Procédé pour faire fonctionner un dispositif de localisation de matériel stérile, comprenant une source d'énergie (101) et une électronique (102), la source d'énergie (101) étant conçue pour alimenter l'électronique (102) en énergie, dans lequel une opération de lavage ou de stérilisation est détectée (400) et au moins une ou plusieurs parties désactivables de l'électronique (102) sont au moins temporairement désactivées pendant l'opération (401), **caractérisé en ce que** la ou les parties désactivées de l'électronique (102) sont rincées pendant l'opération et que la source d'énergie (101) est encapsulée.

10. Procédé selon la revendication 9, **caractérisé en ce qu'**une température est détectée et qu'au moins la ou les parties désactivables de l'électronique (102) sont désactivées lorsque le seuil de température est atteint ou dépassé.

11. Procédé selon la revendication 10, **caractérisé en ce que** les parties désactivables de l'électronique (102) qui ont été désactivées sont activées, notamment à des intervalles temporels réguliers ; il est vérifié si la température est inférieure au seuil de température ; la ou les parties désactivables de l'électronique (102) sont désactivées si la température atteint ou dépasse le seuil de température, sinon les parties activées restent activées.

12. Procédé selon l'une quelconque des revendications 9 à 11, **caractérisé en ce qu'**un signal d'activation au moins pour la ou les parties désactivables de l'électronique (102) est reçu, lesdites parties étant activées lors de la réception du signal d'activation, et/ou **en ce qu'**un signal de désactivation au moins pour la ou les parties désactivables de l'électronique (102) est reçu, lesdites parties étant désactivées lors de la réception du signal de désactivation.

13. Procédé selon l'une quelconque des revendications 9 à 12, **caractérisé en ce que** la ou les parties désactivables de l'électronique (102) ne sont activées qu'après un certain délai suivant la détection de l'opération de stérilisation.

14. Procédé selon l'une quelconque des revendications 9 à 13, **caractérisé en ce que** le dispositif (100) comprend une bobine, en particulier une bobine pour la communication en champ proche, de préférence une étiquette NFC ou une étiquette RFID, une tension induite dans la bobine étant détectée et la ou les parties désactivables de l'électronique (102) étant activées lors de la détection de la tension induite.
